# EUROPEAN PATENT SPECIFICATION

(11) **EP 1 855 628 B1**
(45) Date of publication and mention of the grant of the patent: **18.08.2010**
(21) Application number: 06700953.0
(22) Date of filing: 13.01.2006
(51) Int. Cl.: A61F 5/44

(54) **DISPOSABLE FLUID COLLETION BAG FOR HYGIENIC PURPOSES**
EINWEGSAMMELBEUTEL FÜR HYGIENISCHE ANWENDUNG
SAC JETABLE COLLECTEUR DE FLUIDES BIOLOGIQUES A DES FINS D'HYGIENE

(30) Priority: 28.02.2005 EP 05075477
(43) Date of publication of application: 21.11.2007
(73) Proprietor: Unomedical A/S, 3460 Birkerød (DK)
(72) Inventor: NORDBO, Kalle, Jon, DK-2300 Copenhagen S (DK); GÖRANSSON, Magnus, S-214 21 Malmö (SE); HANSEN, Trygve, Kalf, DK-4040 Jyllinge (DK)
(74) Representative: Dragsted, Helle Rude
(86) International application number: PCT/EP2006/000378
(87) International publication number: WO 2006/089600

(56) References cited:
- EP-A- 0 847 742
- EP-A- 1 473 001
- GB-A- 2 336 830
- US-A- 4 990 145
- US-A- 5 569 225

## Description

The present invention relates to a disposable fluid collection bag, in particular a urine bag of the kind mentioned in the preamble of claim. 1. The document EP-A-0847742 is regarded as the closest prior art.

From EP 0 748 620 B1 and EP 0 847 742 B1, a fluid collection bag for use for hygienic purposes of this kind is known. This type of disposable urine bag disclosed in EP 0 748 620 B1 and EP 0 847 742 B1 comprises a collar having an opening into a plastic pouch having two walls. Between said walls, an non-return valve integrally formed is provided. The non-return flow arrangement consists of three pairs of plastic foil sheets extending down from the opening with increasing lengths, loosely held in place by point welding spots on each pair of foil sheets transversely of the pouch in order to divide the pouch into an upper and a lower part. The upper part is provided with a weld along the edge of the pouch to seal it.

A disadvantage of this urine bag is the limitations in relation to its manufacturing, in particular to the welding of the urinal. It may be complicated to join together eight layers by welding in a fluid tight manner since the welding must join together two pouch walls and six valve layers, i.e. three pairs for forming a non-return flow system below the inlet The side walls are typically made of a first type of plastic material, whereas the non-return flow layers are made of another and/or individually different plastic material. This result in different welding conditions along the bag as the number of layers and the types of materials differ depending on which part of the bag is to be welded. This incurs extra production costs since it must be ensured that the weldings are fluid tight irrespective of foil materials or amount of layers involves. Moreover, these known types of disposable urine bags include a relatively high amount of material, i.e. eight plastic layers in said upper section.

US 5,569,225 and US 4,990,145 disclose a disposable bag for collecting bodily fluids for testing. In this bag design, the trapez-like shaped inlet is succeeded in the inward flow direction with a substantially congruent trapez-shaped flexible valve which overlaps the inlet. However, this valve means does not provide a satisfying prevention of fluid return flow. Therefore, the bag is provided with a hand protection shielding the hand of a user gripping around the test fluid bag. This does not ensure hygienic use of the fluid collection bag. Accordingly, this bag is exclusively designed for collecting small samples of bodily fluids, where the required fluid volume is relatively smell compared to urinary collection bags.

On this basis, it is an object by the present invention to provide a disposable fluid collection bag for hygienic purposes, in particular a urine bag, of the initially mentioned kind, which is simple and inexpensive in manufacture.

This object is achieved by a fluid collection bag wherein the flexible non-return valve means extends into the fluid collection compartment, and that the valve means are mounted directly to the inlet opening collar and wherein the flexible non-return valve means extends into the fluid collection compartment, where the valve means are mounted directly to the inlet opening collar, and that the foil layers of the valve means are having different lengths.

By providing the flexible non-return valve means as a separate element, the non-return valve means may be made in a special flexible foil material since the valve foils need not be welded together with the foils farming the pouch. By mounting the valve means directly to the inlet collar, the pouch is not divided into two compartments since the entire volume of the pouch may constitute the fluid collection compartment, thereby increasing the capacity of the fluid collection bag.

This also means that when emptying the fluid collection bag, the bag may be punctured at its uppermost portion above the fluid level, and the fluid can be poured out through this opening. This is not possible by the hitherto known disposable urine bags due to the integrally formed non-return valve means.

According to the preferred embodiment, the non-return valve means comprises a plurality of foil layers of different lengths, where these foil layers expending from the inlet collar and are joined together to define a general flow direction into the fluid compartment. Moreover, discrete spot weldings are preferably provided at the end sections of each set of foil layers joining all the present the foil layers together. This means that the fluid will open the foils when flowing through in the direction from the inlet to the distal ends of the foils. However, if the a counter flow should occur, this will cause the flow passage between the foil layers to close, and thereby prevent the fluid from escaping the fluid compartment, once the t3uid has passed the flexible foil arrangement of the non-return valve means.

The flexible pouch is preferably made of two flexible foil sheet layers of substantially identical shape joined together along their periphery with the inlet opening collar provided between said foil sheets, and that said sheets are joined to the inlet collar instead of each other at the position of the inlet opening collar of the bag. Whereby, the pouch is made and the inlet may be mounted and sealed in the same welding profess.

In the preferred embodiment, the inlet opening collar is provided centrally on the pouch, which may be substantially rectangular in shape. By providing the inlet centrally on the upper side of the bag, the upper corner regions may be used for handing and emptying, which in turn provides for an easy handling of the bag during use.

The inlet collar is preferably moulded With a flattened configuration, which is operable by pushing at the two opposite folds in the flattened configuration against each other. Various inlet collar features may advantageously be provided.

A bag according to the invention may be provided with outlet means for emptying the fluid in the collection compartment. In a preferred embodiment, the outlet means is provided adjacent the inlet opening of the bag. Various outlet means may be provided.

In an advantageous embodiment of the invention, a test compartment is provided adjacent the outlet means in such a manner that the test compartment is filled with the fluid during the emptying of the pouch for collecting a sample e.g. for further analysis of the collected fluid.

A bag according to the invention may be made of flexible transparent or translucent plastic foil maternal.

By a urinal according to the invention, the advantageous differences include
- that the opening is placed in the middle of the upper part of the urinal and is provided with a spout, which opens and closes with a closure effect known from wallets, but not with two internal tongues, as in the former patent,
- that a tear corner is provided for easy disposal of the urine, this being placed in the upper part of the bag, which facilitates the emptying of the urinal as compared to a positioning in the lower part of the bag, and
- an inner sample pouch is provided near the tear corner for easy stick insertion through the torn corner for sampling the urine.

Apart from the above described embodiments, a further embodiment of a collection bag for collecting bodily fluids could be that the tear corner is provided, with variable width tear ridges to facilitate a slow pouring when taking: samples, or a fast pouring when disposing of the collected urine; and that a lower part of the valve is provided with a urine test strip for easy recording of values, evading the problem of having urine poured over hands and the like during sampling.

In an embodiment of the invention, the inlet opening collar is an inlet tube. The tube may be connected to a catheter. In addition, the fluid compartment may be provided with a drainage tube with a stop valve,

The invention is described in more detail with reference to the accompanying drawings, in which:
- Fig. 1: shows a fluid collection bag according to a preferred embodiment of the invention;
- Fig. 2: shows the flexible foil arrangement in a non-return valve means;
- Fig. 3: is a perspective view of an inlet collar according to the invention;
- Fig. 4: shows the same from a bottom perspective view; and
- Fig. 5 to 7: are schematic views of three embodiments of a fluid collection bag according to the invention with outlet means.

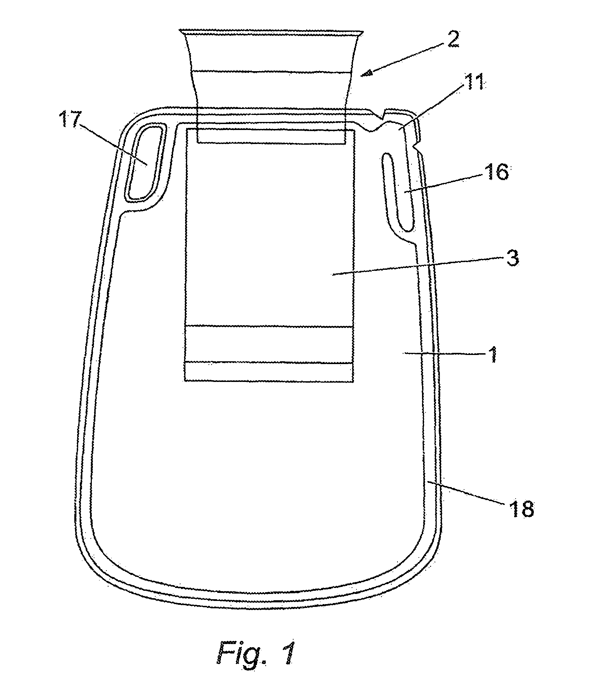

With reference to figure 1, a fluid collection bag according to a preferred embodiment of the invention is shown. The bag comprises a pouch 1, an inlet opening collar 2 and a flexible foil arrangement 3 forming the non-return valve means ensuring that fluid which flows through the inlet 2 and into the pouch 1 cannot escape through the foil arrangement 3. The foil arrangement 3 is mounted directly below the inlet collar 2 and extends into the pouch 1 as it is apparent in fig. 1.

In the pouch 1 a small compartment 16 and a tear strip 11 are formed providing means for fluid outlet in the upper corner adjacent the centrally disposed inlet collar 2. In the opposite upper corner region of the pouch 1 a handling aperture 17 is provided for e.g, hanging the urine bag on a hook or the like and/or for holding the bag during filling and emptying fluids.

The pouch 1 includes two flexible foil layers, e.g. two identically shaped foil sheets as shown in fig. 1, or one foil which is folded. The foils are provided with a weld seam 18 along the periphery of the pouch 1. The non-return valve foil arrangement 3 is welded or otherwise permanently joined to the inlet collar 2. Subsequently, the inlet collar 2 is sandwiched between the foil layers masking up the pouch and welded together with the foil layer to form the inlet of the pouch 1.

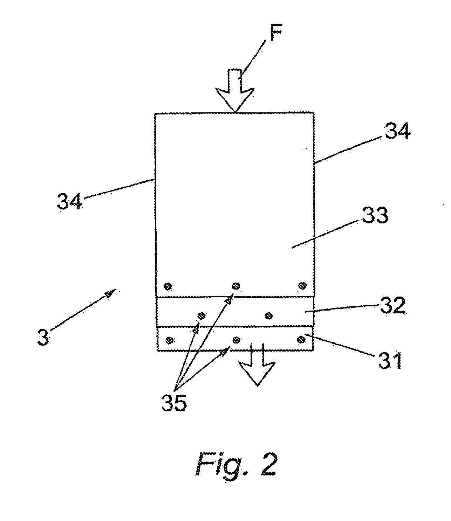

The non-return valve foil arrangement 3 is not welded to the sides of the pouch, as it is the case in the prior art, but extends from the inlet collar and into the fluid compartment of the pouch. The Non-return valve 3, which is shown in fig. 2, is made of six flexible foil layers formed in three pairs 31, 32, 33, where the foil layers 31, 32, 33 are welded along their sides 34 and a spot is welded 35 at the distal end of the foil arrangement to form a directional flow path F through the non-return flexible foil valve arrangement 3. In this embodiment, the inner foil layers may be provided with a length or 10 cm, the pair of middle foil layers having a length of 13 cm and the outermost foil layers having a length of 14 cm. The material of the foil may be PELD, which is a low density polyethylene which is transparent and soft and highly flexible. However, it is realised that other types of flexible foil; materials may be used, including both mono and multilayered foils. The inlet collar is made of a relatively rigid plastic material.

The point weldings 35 are provided in one or more lines, and the lowermost line could extend all the way across and also joining the outermost non-return valve foils 31 to the bag foils forming the pouch 1.

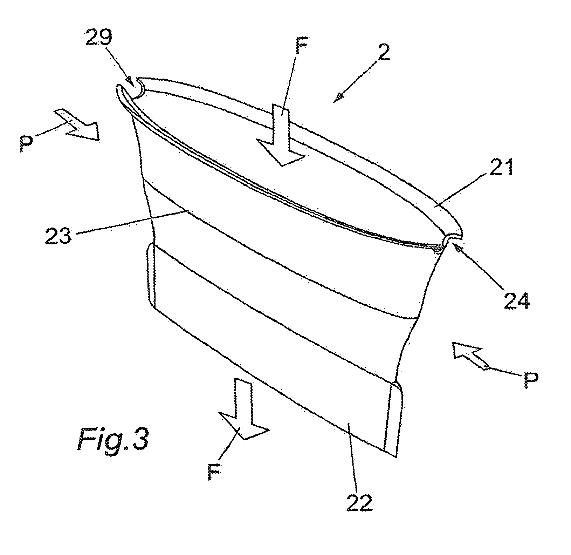

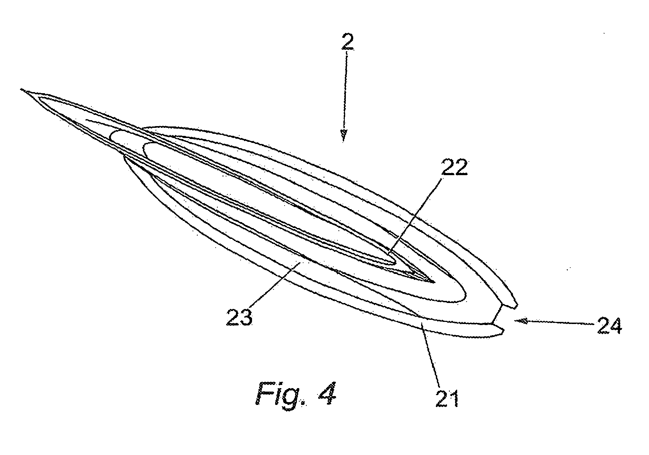

The inlet collar 2 is shown in more detail in figures 3 and 4. The inlet collar 2 is disposed in the central portion of the urine bag, as shown in fig. 1. The inlet collar 2 is made in a resilient plastic material and with a generally flat configuration, which is openable when pressure is applied to the side edges, as indicated by the arrows P. thereby, the two side portions 23 bend outwards and create the flow passage allowing the flow F to pass through the inlet opening. The outermost periphery 23 may be provided with a cut-out 24, 29 at the two corner edges in order to avoid that the inlet collar sides 23 buckle when opening the inset collar 2 by applying the inward pressure P on the folds.

As shown in fig. 3, the inlet opening collar 2 is preferably moulded with an elliptically shaped outermost end periphery 21 and a generally flay openable innermost end periphery 22.

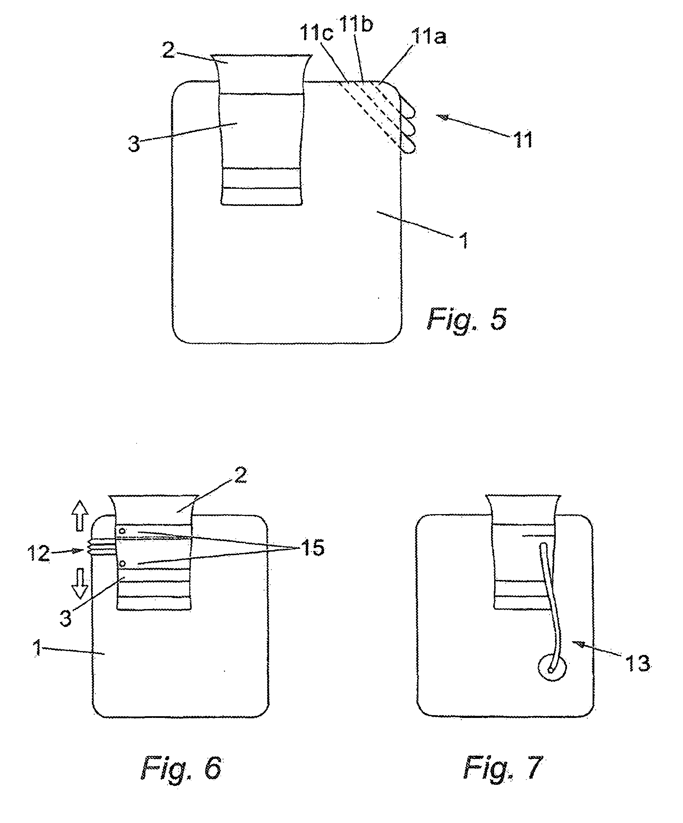

As described with reference to fig. 1, a tear strip 11 may be provided in the corner of the pouch for providing an outlet opening. As shown in fig. 5, anther embodiment could be to provide a multiple of substantially congruently formed tear strips 11a, 11b, 11c in the corner region for providing a plurality of possible sizes of an outlet opening.

Other embodiments of outlet means in a fluid collection bag according to the invention are shown in figures. 6 and 7. For instance as shown in fig. 6, the outlet means may include tearing means 12 for destroying the non-tetum valve 3 as two weld spots 15 are provided joining the flexible non-return valve 3 to the pouch foil, Another embodiment is shown in fig. 7, where a tearing strip 13 is provided in the pouch 1 for providing a by-pass of the non-return valve 3 and allowing the collected fluid two flow from the fluid collection compartment to the inlet opening 1.

The invention is described above with reference to some preferred embodiments. However, it is realised that other embodiments and variations of the fluid collection bag may be provided without departing from the scope of protection defined in the accompanying claims.

## Claims

1. A fluid collection bag for use for hygienic purposes, in particular a disposable urine bag, comprising:
a flexible foil pouch (1) defining a fluid collection compartment (16),
an inlet opening collar (2) mounted in the pouch (1) for allowing fluid being entered into the pouch (1), and
non-return valve means (3) comprising a plurality of flexible foil layers having different lengths (31,32,33) between the inlet opening collar (2) and the fluid collection compartment (16) for preventing the fluid from escaping the fluid collection compartment (16),
**characterise in that**
- the flexible non-return valve means (3) extends into the fluid collection compartment (16) where the valve means (3) are mounted directly to the inlet opening collar (2),
- the inlet collar (2) is a tubular member formed with an openable, flattened configuration in a non-active position having two side portions (23) with two oppositely situated longitudinally oriented folds and an exterior inlet periphery opening (23) and an internal periphery opening with a tapered collar between said two peripheral openings, the tubular opening is established by applying an inwardly oriented pressure to the folds on the outermost periphery (23), and
- the exterior inlet periphery opening (23) is provided with a cut-away (24,29) in connection to the folds for avoiding buckling of the collar (2) when activating the opening (23) by applying an inward pressure on the folds.

2. A fluid collection bag according to claim. 1, wherein the plurality of foil layers of different lengths of the non-return valve means extend from the inlet collar and are joined together to define a general flow direction into the fluid compartment.

3. A fluid collection bag according to claim 2, wherein discrete spot weldings are provided at the end sections of each set of foil layers, joining all the foil layers together.

4. A fluid collection bag according to any of the preceding claims, wherein the flexible pouch is made of two flexible foil sheet layers of substantially identical shape joined together along their periphery with the inlet opening collar provided between said foil sheets, and that said sheets are joined to the inlet collar instead of each other at the position of the inlet opening collar of the bag.

5. A fluid collection bag according to any of the preceding claims, wherein the inlet opening collar is provided centrally on the substantially rectangular shaped pouch.

6. A fluid collection bag according to any of the preceding claims, wherein the inlet opening collar comprises a set of collar members on the periphery of the inlet opening which in a flattened position closes the opening and which by applying pressure on the extreme ends of said collar members causes the members to bend and thereby provide an opening of the inset.

7. A fluid collection bag according to claim 6, wherein the set of openable collar members is provided on the innermost periphery end of the inlet collar.

8. A fluid collection bag according to any of claims 5 to 7, wherein the inlet opening collar is provided with an elliptically shaped outermost end periphery and a generally flat openable innermost end periphery.

9. A fluid collection bag according to any of the preceding claims, wherein the inlet opening collar extends into the fluid compartment.

10. A fluid collection bag according to any of the preceding claims, wherein the inlet opening collar extends out of the periphery of the pouch.

11. A fluid collection bag according to any of the preceding claims, wherein outlet means are provided for emptying the fluid in the collection compartment.

12. A fluid collection bag according to claim 11, wherein the outlet means is provided adjacent the inlet opening of the bag.

13. A fluid collection bag according to claim 11 or 12, wherein the outlet means comprises a tear strip for providing an emptying opening, said tear strip being provided in the corner of the pouch.

14. A fluid collection bag according to claim 13, wherein the outlet means comprises a plurality of substantially congruently formed tear strips, said tear strips are provided in a corner region for providing a plurality of possible sizes of the emptying opening.

15. A fluid collection bag according to claim 11, wherein the outlet means include a tearing strip in the pouch for providing a by-pass of the non-return valve and allowing the collected fluid to flow from the fluid collection compartment to the inlet opening.

16. A fluid collection bag according to claim 11, wherein the outlet means includes tearing means for destroying the non-return valve.

17. A fluid collection bag according to any of the preceding claims, wherein a test compartment is provided adjacent the outlet means in such a manner that the test compartment is filled with the fluid during the emptying of the pouch for collecting a sample e.g. for further analysis of the collected fluid.

18. A fluid collection bag according to any of the preceding claims, wherein the bag is made of flexible transparent plastic foil material.

19. A fluid collection bag according to any of the preceding claims, wherein the bag is made of flexible translucent plastic foil material.

## Patentansprüche

1. Fluidsammelbeutel für die Verwendung bei Hygieneanwendungen, insbesondere ein Urin-Einwegbeutel, enthaltend:
eine flexible Folientasche (1), die ein Fluidsammelabteil (16) bildet,
einen Einlassöffnungskragen (2), der in der Tasche (1) angebracht ist und einen Eintritt des Fluids in die Tasche (1) gestattet, sowie
eine Absperrventileinrichtung (3), die eine Vielzahl flexibler Folienschichten unterschiedlicher Längen (31, 32, 33) zwischen dem Einlassöffnungskragen (2) und dem Fluidsammelabteil (16) enthält um zu verhindern, dass das Fluid aus dem Fluidsammelabteil (16) entweicht,
**dadurch gekennzeichnet, dass**
- sich die flexible Absperrventileinrichtung (3) in das Fluidsammelabteil (16) erstreckt, wobei die Ventileinrichtung (3) direkt an dem Einlassöffnungskragen (2) angebracht ist,
- der Einlasskragen (2) ein röhrenförmiges Element ist, das in einer öffenbaren, abgeflachten Konfiguration in einer nicht aktiven Stellung ausgebildet ist und über zwei Seitenabschnitte (23) mit zwei gegenüberliegend angeordneten, in Längsrichtung ausgerichteten Falzen und eine Außenumfangseinlassöffnung (23) sowie eine Innenumfangsöffnung mit einem konischen Kragen zwischen den beiden Umfangsöffnungen verfügt, wobei die röhrenförmige Öffnung **dadurch** eingerichtet wird, dass ein nach innen gerichteter Druck auf die Falze am äußersten Umfang (23) ausgeübt wird, und
die Außenumfangseinlassöffnung (23) mit einem Ausschnitt (24, 29) in Verbindung mit den Falzen versehen ist, um ein Ausknicken des Kragens (2) zu verhindern, wenn die Öffnung (23) durch Ausüben eines nach innen gerichteten Drucks auf die Falze eingerichtet wird.

2. Fluidsammelbeutel nach Anspruch 1, bei dem sich die Vielzahl von Folienschichten unterschiedlicher Längen der Absperrventileinrichtung von dem Einlasskragen erstrecken und miteinander verbunden sind, um eine allgemeine Flussrichtung in das Fluidabteil zu definieren.

3. Fluidsammelbeutel nach Anspruch 2, bei dem getrennte Punktverschweißungen an den Endabschnitten jeder der Folienschichten vorgesehen sind, die sämtliche Folienschichten miteinander verbinden.

4. Fluidsammelbeutel nach einem der vorhergehenden Ansprüche, bei dem die flexible Tasche aus zwei flexiblen Folienblattschichten im wesentlichen identischer Form besteht, die entlang ihres Umfangs mit dem Einlassöffnungskragen verbunden sind, der zwischen den Folienblättern vorgesehen ist, wobei diese Blätter, anstelle miteinander verbunden zu sein, mit dem Einlasskragen an der Stelle des Einlassöffnungskragens des Beutels verbunden sind.

5. Fluidsammelbeutel nach einem der vorhergehenden Ansprüche, bei dem der Einlassöffnungskragen zentral an der im wesentlichen rechteckigen Tasche vorgesehen ist.

6. Fluidsammelbeutel nach einem der vorhergehenden Ansprüche, bei dem der Einlassöffnungskragen einen Satz von Kragenelementen am Umfang der Einlassöffnung enthält, der in einer abgeflachten Stellung die Öffnung verschließt und durch Ausüben eines Drucks auf die äußersten Enden der Kragenelemente bewirkt, dass sich die Elemente biegen und **dadurch** eine Öffnung an dem Einlass bereitstellen.

7. Fluidsammelbeutel nach Anspruch 6, bei dem der Satz öffenbarer Kragenelemente am innersten Umfangsende des Einlasskragens vorgesehen ist.

8. Fluidsammelbeutel nach einem der Ansprüche 5 bis 7, bei dem der Einlassöffnungskragen mit einem ellipsenförmigen äußersten Endumfang und einem im wesentlichen flachen öffenbaren innersten Endumfang versehen ist.

9. Fluidsammelbeutel nach einem der vorhergehenden Ansprüche, bei dem sich der Einlassöffnungskragen in das Fluidabteil erstreckt.

10. Fluidsammelbeutel nach einem der vorhergehenden Ansprüche, bei dem sich der Einlassöffnungskragen aus dem Umfang der Tasche erstreckt.

11. Fluidsammelbeutel nach einem der vorhergehenden Ansprüche, bei dem eine Auslasseinrichtung vorgesehen ist, um das Fluid in dem Sammelabteil zu entleeren.

12. Fluidsammelbeutel nach Anspruch 11, bei dem die Auslasseinrichtung benachbart der Einlassöffnung des Beutels vorgesehen ist.

13. Fluidsammelbeutel nach Anspruch 11 oder 12, bei dem die Auslasseinrichtung einen Abreißstreifen für die Bereitstellung einer Entleerungsöffnung enthält, wobei der Abreißstreifen an der Ecke der Tasche vorgesehen ist.

14. Fluidsammelbeutel nach Anspruch 13, bei der die Auslasseinrichtung eine Vielzahl im wesentlichen kongruent ausgebildeter Abreißstreifen enthält, wobei diese Abreißstreifen in einem Eckbereich vorgesehen sind, um eine Vielzahl möglicher Größen der Entleerungsöffnung bereitzustellen.

15. Fluidsammelbeutel nach Anspruch 11, bei der die Auslasseinrichtung einen Abreißstreifen in der Tasche enthält, um eine Umgehungsleitung des Absperrventils bereitzustellen und es dem gesammelten Fluid zu gestatten, von dem Fuidsammelabteil zur Einlassöffnung zu fließen.

16. Fluidsammelbeutel nach Anspruch 11, bei dem die Auslasseinrichtung eine Reißeinrichtung zum Zerstören des Absperrventils enthält.

17. Fluidsammelbeutel nach einem der vorhergehenden Ansprüche, bei dem ein Testabteil benachbart der Auslasseinrichtung derart vorgesehen ist, dass das Testabteil mit dem Fluid während des Entleerens der Tasche gefüllt wird, um eine Probe, wie etwa für die weitere Analyse des gesammelten Fluids, zu sammeln.

18. Fluidsammelbeutel nach einem der vorhergehenden Ansprüche, wobei der Beutel aus einem flexiblen, transparenten Kunststofffolienmaterial besteht.

19. Fluidsammelbeutel nach einem der vorhergehenden Ansprüche, wobei der Beutel aus einem flexiblen, transluzenten Kunststofffolienmaterial besteht.

## Revendications

1. Sac de collecte de fluide utilisé à des fins hygiéniques, en particulier un sac à urine jetable, comprenant :
un sachet en feuille flexible (1) définissant un compartiment de collecte de fluide (16),
un collier d'ouverture d'orifice d'entrée (2) monté dans le sachet (1) pour permettre au fluide d'entrer dans le sachet (1), et
des moyens formant clapet anti-retour (3) comprenant une pluralité de couches de feuilles flexibles (31, 32, 33) ayant différentes longueur entre le collier d'ouverture d'orifice d'entrée (2) et le compartiment de collecte de fluide (16) pour empêcher le fluide de s'échapper du compartiment de collecte de fluide (16),
**caractérisé en ce que**
les moyens formant clapet anti-retour (3) s'étendent dans le compartiment de collecte de fluide (16), où les moyens de clapet (3) sont montés directement sur le collier d'ouverture d'orifice d'entrée (2),
le collier d'ouverture d'orifice d'entrée (2) est un élément tubulaire formé avec une configuration ouvrable aplatie dans une position non active ayant deux parties latérales (23) avec deux feuilles orientées longitudinalement opposées l'une à l'autre et une ouverture de périphérie d'orifice d'entrée extérieur (23) et une ouverture de périphérie intérieure avec un collier conique entre lesdites deux ouvertures périphériques, l'ouverture tubulaire est établie en appliquant une pression orientée vers l'intérieur sur les plis sur la périphérie la plus extérieure (23), et
l'ouverture de périphérie d'orifice d'entrée extérieur (23) est pourvue d'une découpe (24, 25) en liaison avec les plis pour éviter le gondolage du collier (2) lors de l'activation de l'ouverture (23) en appliquant une pression vers l'intérieur sur les plis.

2. Sac de collecte de fluide selon la revendication 1, dans lequel la pluralité de couches de feuilles de différentes longueurs des moyens formant clapet anti-retour s'étendent au-delà du collier intérieur et sont jointes ensemble pour définir une direction générale d'écoulement dans le compartiment de fluide.

3. Sac de collecte de fluide selon la revendication 2, dans lequel des soudures en points discrètes sont placées au niveau des sections d'extrémité de chaque série de couches de feuilles, joignant toutes les couches de feuilles ensemble.

4. Sac de collecte de fluide selon l'une quelconque des revendications précédentes, dans lequel le sachet flexible est fait de deux couches de feuilles en feuilles flexibles de forme sensiblement identique jointes ensemble le long de leur périphérie avec le collier d'ouverture d'orifice d'entrée placé entre lesdites feuilles en feuilles, et en ce que lesdites feuilles sont jointes au collier d'orifice d'entrée au lieu d'être jointes ensemble dans la position du collier d'ouverture d'orifice d'entrée du sac.

5. Sac de collecte de fluide selon l'une quelconque des revendications précédentes, dans lequel le collier d'ouverture d'orifice d'entrée est placé centralement sur le sachet de forme sensiblement rectangulaire.

6. Sac de collecte de fluide selon l'une quelconque des revendications précédentes, dans lequel le collier d'ouverture d'orifice d'entrée comprend une série d'éléments de collier sur la périphérie de l'ouverture d'orifice d'entrée qui dans une position aplatie ferme l'ouverture et qui en appliquant une pression sur les extrémités extrêmes desdits éléments de collier font se courber les éléments et donc permettent une ouverture de l'orifice d'entrée.

7. Sac de collecte de fluide selon la revendication 6, dans lequel la série d'éléments de collier ouvrables est placée sur l'extrémité de périphérie la plus vers l'intérieur du collier d'orifice d'entrée.

8. Sac de collecte de fluide selon l'une quelconque des revendications 5 à 7, dans lequel le collier d'ouverture d'orifice d'entrée est pourvu d'une périphérie d'extrémité la plus extérieure formée elliptiquement et d'une périphérie d'extrémité la plus vers l'intérieur ouvrable globalement plate.

9. Sac de collecte de fluide selon l'une quelconque des revendications précédentes, dans lequel le collier d'ouverture d'orifice d'entrée s'étend dans le compartiment de fluide.

10. Sac de collecte de fluide selon l'une quelconque des revendications précédentes, dans lequel le collier d'ouverture d'orifice d'entrée s'étend hors de la périphérie du sachet.

11. Sac de collecte de fluide selon l'une quelconque des revendications précédentes, dans lequel des moyens formant orifice de sortie sont prévus pour vider le fluide dans le compartiment de collecte.

12. Sac de collecte de fluide selon la revendication 11, dans lequel les moyens formant orifice de sortie sont placés adjacents à l'ouverture d'orifice d'entrée du sac.

13. Sac de collecte de fluide selon la revendication 11 ou 12, dans lequel les moyens formant orifice de sortie comprennent une bande déchirable pour fournir une ouverture de vidage, ladite bande déchirable étant placée dans le coin du sachet.

14. Sac de collecte de fluide selon la revendication 13, dans lequel les moyens formant orifice de sortie comprennent une pluralité de bandes déchirables formées sensiblement congrument, lesdites bandes déchirables sont placées dans une région de coin pour fournir une pluralité de tailles possibles de l'ouverture de vidage.

15. Sac de collecte de fluide selon la revendication 11, dans lequel les moyens formant orifice de sortie incluent une bande de déchirement dans le sachet pour fournir une dérivation au clapet anti-retour et permettre au fluide collecté de s'écouler du compartiment de collecte de fluide vers l'ouverture d'orifice d'entrée.

16. Sac de collecte de fluide selon la revendication 11, dans lequel les moyens formant orifice de sortie incluent des moyens de déchirement pour détruire le clapet anti-retour.

17. Sac de collecte de fluide selon l'une quelconque des revendications précédentes, dans lequel un compartiment de test est placé adjacent aux moyens formant orifice de sortie de telle manière que le compartiment de test est rempli avec le fluide pendant le vidage du sachet pour collecter un échantillon par exemple pour une analyse ultérieure du fluide collecté.

18. Sac de collecte de fluide selon l'une quelconque des revendications précédentes, dans lequel le sac est fait d'un matériau de feuille plastique transparente flexible.

19. Sac de collecte de fluide selon l'une quelconque des revendications précédentes, dans lequel le sac est fait d'un matériau de feuille plastique translucide flexible.
